## Europäisches Patentamt
## European Patent Office
## Office européen des brevets

(19)

(11) Numéro de publication: **0 005 100**
**B1**

(12)

## FASCICULE DE BREVET EUROPEEN

(45) Date de publication du fascicule de brevet: **22.07.81**

(51) Int. Cl.³: **C 07 D 301/12,**
**C 07 J 71/00**

(21) Numéro de dépôt: **79400223.8**

(22) Date de dépôt: **05.04.79**

(54) **Nouveau réactif d'époxydation et son application à l'époxydation de composés organiques acycliques, cycliques ou polycycliques comportant au moins une insaturation éthylénique.**

(30) Priorité: **19.04.78 FR 7811517**

(43) Date de publication de la demande:
**31.10.79 Bulletin 79/22**

(45) Mention de la délivrance du brevet:
**22.07.81 Bulletin 81/29**

(84) Etats Contractants Désignés:
**BE CH DE FR GB IT NL SE**

(56) Documents cités:
**FR - A - 2 149 428**
**FR - A - 2 201 287**

(73) Titulaire: **ROUSSEL-UCLAF**
**102, route de Noisy Boîte postale no.9**
**F-93230-Romainville (FR)**

(72) Inventeur: **Costerousse, Germain**
**10 rue des réservoirs**
**F-94410 Saint-Maurice (FR)**
Inventeur: **Teutsch, Jean-Georges**
**Résidence Lavoisier bat. 3 3, rue Lavoisier**
**F-93500 Pantin (FR)**

(74) Mandataire: **Tonnellier, Marie-José et al,**
**102, route de Noisy Boîte postale no 9**
**F-93230 Romainville (FR)**

Courier Press, Leamington Spa, England.

Nouveau réactif d'époxydation et son application à l'époxydation de composés organiques acycliques,
cycliques ou polycycliques comportant au moins une insaturation éthylénique

La présente invention concerne un nouveau réactif d'époxydation et son application à l'époxydation de composés organiques acycliques, cycliques ou polycycliques comportant au moins une insaturation éthylénique.

L'invention a pour objet, à titre de réactif d'époxydation, l'eau oxygénée utilisée en présence d'hexachloroacétone.

On connaissait déjà de nombreux réactifs d'époxydation, notamment l'eau oxygénée concentrée en solution alcaline et les peracides comme les acides peracétique, perphtalique et métachloroperbenzoïque. Par rapport à ces réactifs, l'eau oxygénée, utilisée en présence d'hexachloroacétone, présente l'avantage considérable de permettre des réactions plus spécifiques et de pouvoir être utilisée en milieu neutre.

On connaissait également l'hydroperoxyde d'hexafluoroacétone et l'eau oxygénée en présence d'hexafluoroacétone en tant que réactif d'époxydation (voir brevets français 2 201 187 et 2 149 428).

Il s'agit là de réactifs de laboratoire tout à fait excellents, et l'hydroperoxyde d'hexafluoroacétone notamment permet d'obtenir des réactions très spécifiques.

Malheureusement, ces réactifs sont extrêmement coûteux. L'hexafluoroacétone est, en effet, un produit très cher qui a d'ailleurs pratiquement disparu du marché mondial en tant que tel, et est maintenant vendu principalement sous forme de sesquihydrate. L'hydroperoxyde d'hexafluoroacétone, préparée à partir de perhydrol et d'hexafluoroacétone est également, a fortiori, un produit coûteux. Pour ces raisons, ni l'eau oxygénée en présence d'hexafluoroacétone, ni l'hydroperoxyde d'hexafluoroacétone ne sont des réactifs valables sur le plan industriel, d'autant que, par exemple, il faut utiliser de 1 à 2 moles d'hydroperoxyde d'hexafluoroacétone par mole de composé à époxyder pour obtenir de bons rendements.

Le nouveau réactif d'époxydation, objet de la présente invention est lui très intéressant sur le plan industriel, c'est en effet un réactif bon marché, qui permet d'obtenir des réactions très sélectives avec d'excellents rendements.

Il s'agit donc d'abord d'un réactif bon marché: il n'est pas nécessaire, en effet, d'utiliser de grandes quantités d'hexachloroacétone, des quantités catalytiques suffisent.

Par quantité catalytique d'hexachloroacétone, on entend des quantités molaires d'hexachloroacétone, inférieures au quart des quantités molaires des composés à époxyder.

On peut en effet obtenir de très bons rendements d'époxydation en utilisant de faibles quantités d'hexachloroacétone, par exemple, en utilisant des quantités molaires d'hexachloroacétone inférieures au quart des quantités molaires des composés à époxyder.

La partie expérimentale exposée ci-dessous montre clairement que l'on obtient d'excellents rendements d'époxydation de stéroïdes, en utilisant des quantités molaires d'hexachloroacétone égales au cinquième ou au dizième des quantités molaires des stéroïdes à époxyder.

Le réactif de l'invention est de plus tout à fait inattendu tant dans sa nature que dans les quantités à employer.

En effet dans le brevet français 2 149 428 il est indiqué que l'hexachloroacétone a été trouvée inefficace en tant que catalyseur d'époxydation à l'eau oxygénée et que les composés utilisables possèdent au moins un atome de fluor. L'art antérieur détournait donc l'homme de métier du choix de l'hexachloroacétone en tant que catalyseur d'époxydation à l'eau oxygénée.

Dans le brevet français 2 201 187 est décrite l'application de l'hydroperoxyde d'hexafluoroacétone à l'époxydation des composés organiques insaturés.

L'hydroperoxyde d'hexafluoroacétone est préparé au préalable en faisant barboter l'hexafluoroacétone dans une solution d'eau oxygénée concentrée.

Pour obtenir l'hydroperoxyde d'hexafluoroacétone, il faut faire réagir des quantités équimoléculaires d'hexafluoroacétone et d'eau oxygénée. Le brevet français cité enseigne également que, dans un mode de réalisation préféré du procédé de l'invention, on utilise de 1 à 2 moles d'hydroperoxyde d'hexafluoroacétone par mole de composés à époxyder.

On pouvait donc s'attendre, à ce qu'il faille utiliser des quantités équimoléculaires d'eau oxygénée, d'hexachloroacétone et de composés à époxyder pour obtenir des rendements d'époxydation convenables. Il n'en est rien et la partie expérimentale exposée ci-après montre très clairement que l'on obtient d'excellents rendements d'époxydation en utilisant des quantités catalytiques d'hexafluoroacétone.

Le réactif de l'invention, à savoir l'eau oxygénée en présence d'hexachloroacétone, n'est donc pas évident, d'autant que des quantités catalytiques d'hexachloroacétone sont suffisantes.

L'invention a plus particulièrement pour objet, à titre de réactif d'époxydation, l'eau oxygénée utilisée en présence d'une quantité catalytique d'hexachloroacétone.

Le réactif de l'invention présente également l'avantage de permettre des réactions très sélectives avec d'excellents rendements: il permet notamment d'époxyder sélectivement et avec de très bons rendements une seule double liaison dans le cas de composés en comportant plusieurs. C'est ainsi, par exemple, que si l'on époxyde des 5(10) 9(11) estradiènes, on obtient uniquement des 5(10) époxy avec un rendement presque quantitatif, alors qu'au moyen de réactifs d'époxydation usuels comme l'acide m-chloroperbenzoïque, on obtient des mélanges de dérivés 5(10) et 9(11) époxy (voir L. NEDELEC Bull. Soc. Chim. 70, n°7, 2548).

L'invention a également pour objet une application du réactif de l'invention, caractérisée en ce qui l'on soumet un composé organique acyclique, cyclique ou polycyclique, portant au moins une insaturation éthylénique, à l'action dudit réactif d'époxydation et obtient ainsi un composé époxydé.

Le réactif de l'invention permet d'époxyder tout composé comportant une double liaison. Comme composé linéaire, on peut citer l'éthylène et les composés éthyléniques en général. Comme composés monocycliques, on peut citer notamment tous les cycles à cinq, six ou sept chaînons, par exemple, le cyclohexène ou encore les dérivés terpéniques. Comme composés polycycliques, on peut citer de préférence les composés stéroïdes et l'invention a particulièrement pour objet l'application du réactif de l'invention à l'époxydation des stéroïdes.

L'invention a notamment pour objet l'application caractérisée en ce que l'on fait réagir le réactif de l'invention sur un stéroïde portant un groupement cétal en position 3, at une insaturation éthylénique en 5(6) et obtient le stéroïde correspondant portant un groupement cétal en position 3 et un groupement époxy en position 5(6) sous forme d'un mélange d'isomères $5\alpha$, $6\alpha$ et $5\beta$, $6\beta$-époxy que l'on sépare, si désiré, en chacun des isomères.

L'invention a également pour objet l'application, caractérisée en ce que l'on fait réagir le réactif de l'invention sur un stéroïde portant un groupement cétal en position 3, et deux insaturations éthyléniques en 5(10) et 9(11) et obtient ainsi un stéroïde portant un groupement cétal en position 3, une insaturation éthylénique en 9(11) et un groupement époxy en 5(10) sous forme d'un mélange d'isomères $5\alpha(10\alpha)$ et $5\beta(10\beta)$ époxy que l'on sépare, si désiré, en chacun des isomères, ou encore l'application caractérisée en ce que l'on fait réagir le réactif de l'invention sur un stéroïde portant un groupement alcoyloxy en position 3, et deux insaturations éthyléniques en 5(10) et 9(11) et obtient ainsi un stéroïde portant un groupement alcoyloxy en position 3, une insaturation éthylénique en 9(11) et un groupement époxy en 5(10) sous forme d'un mélange d'isomères $5\alpha(10\alpha)$-époxy et $5\beta(10\beta)$-époxy que l'on sépare, si désiré, en chacun des isomères.

Dans un mode de réalisation préférée des applications du réactif de l'invention, on utilise l'hexachloroacétone en quantité catalytique, on opère à une température comprise entre −10°C et 30°C — et de préférence entre −5°C et +5°C, au sein d'un solvant chloré comme, par exemple, le chlorure de méthylène ou le chloroforme, et éventuellement en présence d'une base tertiaire comme la pyridine.

Les isomères obtenus lors de la mise en oeuvre du procédé, peuvent être séparés selon les méthodes usuelles de cristallisation et de chromatographie.

L'invention a naturellement tout spécialement pour objet les applications du réactif de l'invention décrites dans les exemples suivants qui illustrent l'invention sans toutefois la limiter.

### Exemple 1
17,20,20,21-bis [méthylène (bis oxy)] $5\alpha,6\alpha$-époxy 3,3-(éthylène bis oxy) $16\alpha$-méthyl pregnan 11-one.

On introduit sous atmosphère d'azote 1,5 g de 17,20,20,21-bis [méthylène (bis oxy)] 3,3-(éthylène bis oxy) $16\alpha$-méthyl pregn 5-èn 11-one dans 9 cm3 de chlorure de méthylène. On refroidit la solution obtenue à 0°C et ajoute 0,06 cm3 d'hexachloroacétone, puis 0,15 cm3 d'eau oxygénée à 85%. On agite à 20°C sous atmosphère d'azote, pendant 72 heures, ajoute à nouveau 0,06 cm3 d'hexachloroacétone et maintient à nouveau sous agitation pendant 24 heures. On verse le mélange réactionnel dans une solution renfermant 30 cm3 d'une solution de thiosulfate de sodium 0,5 M et 20 g de glace. On extrait au chlorure de méthylène, lave à l'eau, sèche sur sulfate de sodium et obtient 1,7 g d'un produit que l'on empâte à l'éther isopropylique et que l'on sèche. On obtient ainsi 1,24 g du produit recherché fondant à 248°C.

*Spectre RMN* (CDCl$_3$)
$H_6$ à 169—172,5 Hz

### Exemple 2
3,3-20,20-bis(éthylène bis oxy) $5\alpha$, $10\alpha$-époxy $16\alpha$-méthyl 19-nor pregna 9(11)-ène

On introduit 2 g de 3,3-20,20-bis(éthylène bis oxy) $16\alpha$-méthyl 19-nor pregna 5(10) 9(11)-diène dans 9 cm3 de chlorure de méthylène renfermant une trace de pyridine.

On refroidit à 0°C la solution, ajoute 0,075 cm3 d'hexachloroacétone puis, sous agitation, 0,82 cm3 d'eau oxygénée à 30%. Après 24 heures à 0°C, on verse dans une solution glacée de thiosulfate de sodium renfermant une trace de pyridine, extrait au chlorure de méthylène lave la phase organique à l'aide d'une solution de thiosulfate de sodium, sèche et concentre à sec.

On obtient 2,1 g de produit attendu.

*Spectre RMN* (CDCl$_3$)
1,04 à 1,12 ppm (16$\alpha$-méthyl), 1,32 ppm (—CH$_3$ en 21), 3,9 ppm (cétals), 5,77 et 5,94 ppm (H en 11).

### Exemple 3
3,3-diméthoxy $5\alpha$, $10\alpha$-époxy estr-9(11)-ène 17-one

On introduit 210 g de 3,3-diméthoxy estra-5(10), 9(11)-diène 17-one dans 1050 cm3 de chlorure de méthylène renfermant 1 cm3 de pyridine.

On ajoute 10,5 cm3 d'hexachloroacétone, puis 55 cm3 d'eau oxygénée à 50% à la solution, puis agite pendant 24 heures à +16°C et verse dans une solution aqueuse de thiosulfate de sodium. On extrait au chlorure de méthylène, lave les extraits à l'eau, sèche et concentre à sec. On obtient 246 g de produit attendu.

## Revendications

1. A titre de réactif d'époxydation, l'eau oxygénée utilisée en présence d'hexachloroacétone.

2. A titre de réactif d'époxydation, l'eau oxygénée utilisée en présence d'une quantité catalytique d'hexachloroacétone.

3. Application du réactif d'époxydation défini à la revendication 1 ou 2, caractérisée en ce que l'on soumet un composé organique acyclique, cyclique ou polycyclique portant au moins une insaturation éthylénique, à l'action dudit réactif d'époxydation et obtient ainsi un composé époxydé.

4. Application selon la revendication 3, caractérisé en ce que l'on fait réagir ledit réactif d'époxydation sur un stéroïde portant au moins une insaturation éthylénique et obtient ainsi un stéroïde époxydé.

5. Application selon la revendication 4, caractérisée en ce que l'on fait réagir ledit réactif sur un stéroïde portant un groupement cétal en 3, une insaturation éthylénique en 5(6) et obtient le stéroïde correspondant portant un groupement cétal en position 3 et un groupement époxy en position 5(6) sous forme d'un mélange d'isomères $5\alpha$, $6\alpha$, et $5\beta$, $6\beta$-époxy que l'on sépare, si désiré, en chacun des isomères.

6. Application selon la revendication 4, caractérisée en ce que l'on fait réagir ledit réactif sur un stéroïde portant un groupement cétal en position 3, et deux insaturations éthyléniques en 5(10) et 9(11) et obtient ainsi un stéroïde portant un groupement cétal en position 3, une insaturation éthylénique en 9(11) et un groupement époxy en 5(10) sous forme d'un mélange d'isomères $5\alpha(10\alpha)$ et $5\beta(10\beta)$-époxy que l'on sépare, si désiré, en chacun des isomères.

7. Application selon la revendication 4, caractérisée en ce que l'on fait réagir ledit réactif sur un stéroïde portant un groupement alcoyloxy en position 3, et deux insaturations éthyléniques en 5(10) et 9(11) et obtient ainsi un stéroïde portant un groupement alcoyloxy en position 3, une insaturation éthylénique en 9(11) et un groupement époxy en 5(10) sous forme d'un mélange d'isomères $5\alpha(10\alpha)$-époxy et $5\beta(10\beta)$-époxy que l'on sépare, si désiré, en chacun des isomères.

8. Application selon l'une quelconque des revendications 3 à 7, caractérisée en ce que l'on opère à une température comprise entre −10°C et 30°C et de préférence entre −5°C et +5°C.

9. Application selon l'une quelconque des revendications 3 à 8, caractérisée en ce que l'on opère au sein d'un solvant chloré comme, par exemple, le chlorure de méthylène ou le chloroforme.

## Claims

1. As epoxidation reagent, hydrogen peroxide used in the presence of hexachloroacetone.

2. As epoxidation reagent, hydrogen peroxide used in the presence of a catalytic amount of hexachloroacetone.

3. Use of the epoxidation reagent defined in Claim 1 or 2, characterised in that an acyclic, cyclic or polycyclic organic compound bearing at least one ethylenic unsaturation is subjected to the action of the said epoxidation reagent and an epoxidised compound is thus obtained.

4. Use according to Claim 3, characterised in that the said epoxidation reagent is reacted with a steroid bearing at least one ethylenic unsaturation, and an epoxidised steroid is thus obtained.

5. Use according to Claim 4, characterised in that the said reagent is reacted with a steroid bearing a ketal group at 3 and an ethylenic unsaturation at 5(6), and the corresponding steroid bearing a ketal group at position 3 and an epoxy group at position 5(6) is obtained in the form of a mixture of $5\alpha$, $6\alpha$ and $5\beta$, $6\beta$-epoxy isomers which are separated, if desired, into each of the isomers.

6. Use according to Claim 4, characterised in that the said reagent is reacted with a steroid bearing a ketal group at position 3 and two ethylenic unsaturations at 5(10) and 9(11), and a steroid bearing a ketal group at position 3, an ethylenic unsaturation at 9(11) and an epoxy group at 5(10) is thus obtained in the form of a mixture of $5\alpha(10\alpha)$ and $5\beta(10\beta)$-epoxy isomers which are separated, if desired, into each of the isomers.

7. Use according to Claim 4, characterised in that the said reagent is reacted with a steroid bearing an alkyloxy group at position 3 and two ethylenic unsaturations at 5(10) and 9(11), and a steroid bearing an alkyloxy group at position 3, an ethylenic unsaturation at 9(11) and an epoxy group at 5(10) is thus obtained in the form of a mixture of $5\alpha(10\alpha)$-epoxy and $5\beta(10\beta)$-epoxy isomers which are separated, if desired, into each of the isomers.

8. Use according to any one of Claims 3 to 7, characterised in that work is carried out at a temperature included between −10°C and 30°C and preferably between −5°C and +5°C.

9. Use according to any one of Claims 3 to 8, characterised in that work is carried out in a chlorinated solvent such as, for example, methylene chloride or chloroform.

**Patentansprüche**

1. Als Epoxydationsreagens Wasserstoffperoxyd, das in Anwesenheit von Hexachloraceton verwendet wird.

2. Als Epoxydationsreagens Wasserstoffperoxyd, das in Anwesenheit einer katalytischen Menge Hexachloraceton verwendet wird.

3. Verwendung des in Anspruch 1 oder 2 definierten Epoxydationsreagens, dadurch gekennzeichnet, daß man eine acyclische, cyclische oder polycyclische organische Verbindung mit zumindest einer äthylenischen Unsättigung der Einwirkung des Epoxydationsreagens unterzieht und so eine epoxydierte Verbindung erhält.

4. Verwendung gemäß Anspruch 3, dadurch gekennzeichnet, daß man das Epoxydationsreagens auf ein Steroid einwirken läßt, das zumindest eine äthylenische Unsättigung enthält und so ein epoxydiertes Steroid erhält.

5. Verwendung gemäß Anspruch 4, dadurch gekennzeichnet, daß man das Reagens auf ein Steroid einwirken läßt, das in 3-Stellung eine Ketalgruppe und in 5(6)-Stellung eine äthylenische Unsättigung enthält und das entsprechende Steroid mit einer Ketalgruppe in 3-Stellung und einer Epoxygruppe in 5(6)-Stellung in Form eines Gemisches der $5\alpha,6\alpha$- und $5\beta,6\beta$-Epoxyisomeren erhält, das man gewünschtenfalls in die einzelnen Isomeren auftrennt.

6. Verwendung gemäß Anspruch 4, dadurch gekennzeichnet, daß man das Reagens auf ein Steroid mit einer Ketalgruppe in 3-Stellung und zwei äthylenischen Unsättigungen in 5(10)-Stellung und 9(11)-Stellung einwirken läßt und so ein Steroid mit einer Ketalgruppe in 3-Stellung, einer äthylenischen Unsättigung in 9(11)-Stellung und einer Epoxygruppe in 5(10)-Stellung in Form eines Gemisches der $5\alpha(10\alpha)$- und $5\beta(10\beta)$-Epoxyisomeren erhält, das man gewünschtenfalls in die einzelnen Isomeren auftrennt.

7. Verwendung gemäß Anspruch 4, dadurch gekennzeichnet, daß man das Reagens auf ein Steroid mit einer Alkyloxygruppe in 3-Stellung und zwei äthylenischen Unsättigungen in 5(10)- und 9(11)-Stellung einwirken läßt und so ein Steroid mit einer Alkyloxygruppe in 3-Stellung, einer äthylenischen Unsättigung in 9(11)-Stellung und einer Epoxygruppe in 5(10)-Stellung in Form eines Gemisches der $5\alpha(10\alpha)$- und $5\beta(10\beta)$-Epoxyisomeren erhält, das man gewünschtenfalls in die einzelnen Isomeren auftrennt.

8. Verwendung gemäß einem der Ansprüche 3 bis 7, dadurch gekennzeichnet, daß man bei einer Temperatur zwischen −10°C und 30°C und vorzugsweise zwischen −5°C und +5°C arbeitet.

9. Verwendung gemäß einem der Ansprüche 3 bis 8, dadurch gekennzeichnet, daß man in dem Medium eines chlorierten Lösungsmittels, wie zum Beispiel Methylenchlorid oder Chloroform, arbeitet.